# EUROPEAN PATENT SPECIFICATION

(11) **EP 1 657 988 B1**
(45) Date of publication and mention of the grant of the patent: **22.10.2008**
(21) Application number: 04731254.1
(22) Date of filing: 05.05.2004
(51) Int. Cl.: A41D 13/12

(54) **GARMENT FOR PATIENTS IN ENDOSCOPY**
KLEIDUNGSSTÜCK FÜR ENDOSKOPIEPATIENTEN
VETEMENT POUR PATIENTS SUBISSANT UNE ENDOSCOPIE

(30) Priority: 10.06.2003 IL 15638103
(43) Date of publication of application: 24.05.2006
(73) Proprietor: SIGHTLINE TECHNOLOGIES LTD, Haifa 31905 (IL)
(72) Inventor: GOLAN, Salman, 30200 Tirat Hacarmel (IL); SHEZIFI, Omer, 34792 Haifa (IL); BAR-OR, Jacob, 34655 Haifa (IL); AIZENFELD, Amram, 19245 (IL)
(74) Representative: Moore, Barry
(86) International application number: PCT/IL2004/000372
(87) International publication number: WO 2004/107889

(56) References cited:
- US-A- 4 446 575
- US-A- 4 930 161
- US-A- 4 953 566

## Description

### FIELD OF THE INVENTION

The present invention relates generally to garments for use in medical procedures, and specifically to garments to be worn by patients during endoscopy.

### BACKGROUND OF THE INVENTION

A patient undergoing endoscopic examination of the rectum and/or colon must generally lie on an examination table with the lower part of his or her body bared, or at best partly covered by a loose drape. Needless to say, this situation is uncomfortable and embarrassing for the patient. In addition, fluids commonly leak out through the patient's anus during the examination, wetting the patient's body and the examining table.

Examples of known medical examination garments include those described in US4930161 which discloses a garment having a trunk which has front and rear portions, and a waist region where an elastic waist-band is located. The trunk front portion has a rectangular opening intermediate the crotch and the waist region. A front closure with fasteners is provided for covering, partially uncovering and uncovering the front opening. US4953566 discloses a disposable body wrap configured to be worn by a person in the manner of a diaper while the person is undergoing rectal examination. US4446575 discloses a pair of hospital shorts which include a flap secured at its lower end to the rear portion just below an opening in the shorts and which contains a fastener at the opposite end of the flap adapted to be removably fastened to a fastener disposed on the rear portion above the opening whereby the flap covers the opening and can be unfastened to provide access to the patient's anal area.

### SUMMARY OF THE INVENTION

Embodiments of the present invention provide a novel garment to be worn by patients undergoing endoscopic examination. The garment is typically made of absorbent fabric, which is shaped to fit over the pelvic area of the patient in the manner of short pants or a diaper. An aperture is cut through the garment in the area of a body orifice, such as the anus, vagina or urethra, through which the endoscope is to be inserted into the patient's body. The aperture permits access to the orifice for insertion of the endoscope therethrough, while the patient is wearing the garment. Thus, the patient's modesty and comfort are protected during the examination. Fluids that leak out of the orifice are typically absorbed by the garment.

An endoscope retainer is fixed to the garment in the area of the aperture. The physician inserts the endoscope through the retainer into the patient's body orifice. As the physician advances the endoscope into the body, the retainer holds the endoscope in position. Thus, the physician may, when necessary, remove both hands from the endoscope to perform other operations, without requiring an assistant to hold the endoscope in the meanwhile.

There is therefore provided, in accordance with the present invention, an endoscopy apparatus according to claim 1 with embodiments provided in dependent claims.

In a disclosed embodiment, the garment includes a sheet of material, having front and rear portions joined by a central portion, which is cut to fit between the legs, and one or more fasteners, which are adapted to fix the front and rear portions together so as to cover the pelvic area.

The system includes an endoscope retaining device, which is fastened within the aperture so as to hold the endoscope in place when the endoscope is inserted through the body orifice. Typically, the endoscope retaining device includes a rigid material having a bore therethrough, wherein the bore is sized to fit snugly around the endoscope, and wherein the garment includes a fastening assembly for fixing the rigid material within the third hole.

In a disclosed embodiment, the endoscope is adapted to be inserted into an anus of the patient, and the aperture in the garment is sized and located to permit insertion of the endoscope into the anus.

The present invention will be more fully understood from the following detailed description of the embodiments thereof, taken together with the drawings in which:

### BRIEF DESCRIPTION OF THE DRAWINGS

Figs. 1A and 1B are schematic, pictorial, front and rear illustrations of a garment worn by a patient during endoscopy, in accordance with an embodiment of the present invention; and
Fig. 2 is a schematic top view of a fabric pattern used in producing the garment of Figs. 1A and 1B.

### DETAILED DESCRIPTION OF EMBODIMENTS

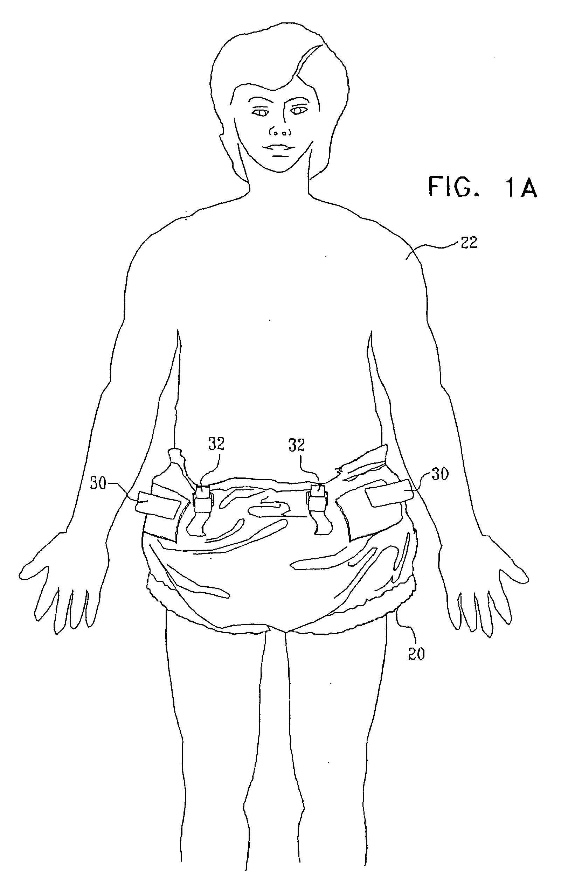

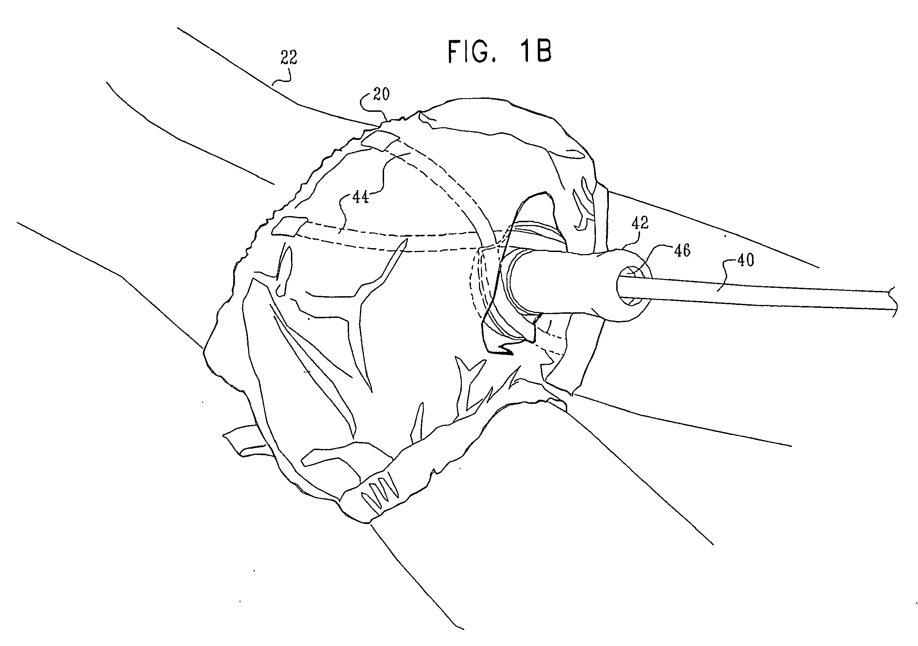

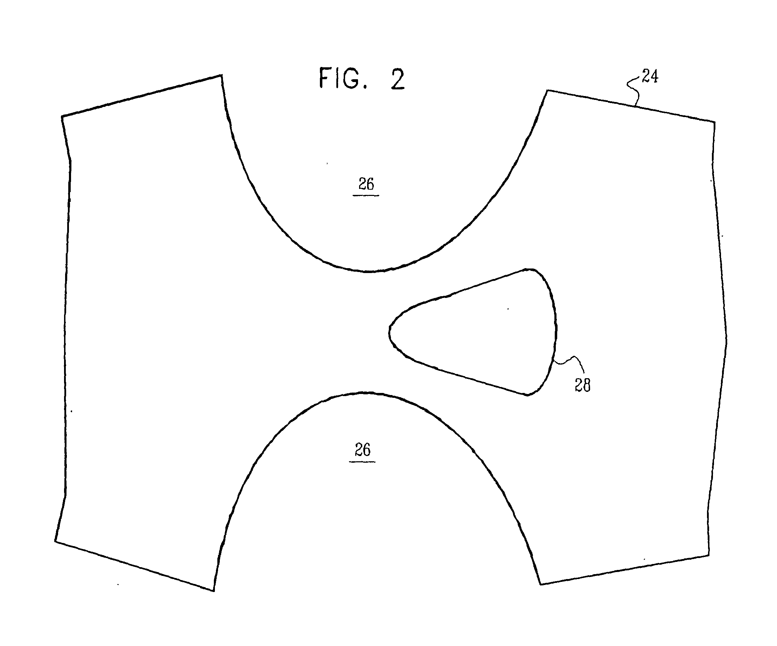

Reference is now made to Figs. 1A, 1B and 2, which schematically illustrate a garment 20 worn by a patient 22 during endoscopy, in accordance with an embodiment of the present invention. Figs. 1A and 1B are pictorial front and rear views, respectively. Fig. 2 is a schematic top view of a fabric pattern 24 used in producing garment 20. The pattern has the general form of a diaper, with leg openings 26 on either side of the central portion of the fabric, and (unlike a conventional diaper) an anal aperture 28. The fabric used to produce garment 20 typically comprises absorbent cloth or paper, possibly with an outer waterproof coating, in order to absorb any fluids that leak out of the patient's anus during the endoscopic examination, and to prevent the fluids from wetting the examining table and other areas of the patient's skin or clothing. Fasteners 30, typically Velcro™ or adhesive fasteners, are used to fix the front and rear portions of garment 20 together around the patient's pelvic region. Straps 32 may be used to adjust the garment for a snug fit.

Alternatively, garment 20 may have the form of short pants, rather than a diaper, with elastic to hold the pants around the patient's waist and thighs.

As shown in Fig. 1B, during the endoscopic examination, the physician inserts an endoscope 40, such as a colonoscope, through aperture 28 into the anus of patient 22. Optionally, the endoscope is inserted through an endoscope retainer 42, which is held in place adjacent to the anus. In the pictured embodiment, retainer 42 is held by a fastening assembly, such as a system of straps 44, which are provided for this purpose as a part of garment 20. Alternatively, retainer 42 may be sewn, glued, clipped or fastened to garment 20 by any other suitable means known in the art. Still further alternatively, excluded from the scope of the invention, aperture 28 may be made sufficiently small and stiff to serve on its own as the endoscope retaining device, without the use of an additional retaining element such as retainer 42.

Retainer 42 typically comprises a rigid plastic material, with a central bore 46 that is preferably sized to fit snugly around endoscope 40. The fit is loose enough so that the physician can insert and withdraw endoscope 40 through bore 46 without substantial resistance, but still tight enough so that when the physician releases his grip on endoscope 40, the endoscope stays in place and does not begin to slide outward. Retainer 42 thus obviates the need for a nurse or other assistant to hold the endoscope while the doctor performs other operations, such as a biopsy.

After the endoscopic examination is finished, garment 20 and retainer 42 are typically removed and discarded.

Although the embodiment described above relates specifically to endoscopic examination of the rectal area and colon, garment 20 may similarly be adapted for use in endoscopic examination made through other body passages, such as the vagina and urethra. It will thus be appreciated that the embodiments described above are cited by way of example, and that the present invention is not limited to what has been particularly shown and described hereinabove. The scope of the present invention includes the various features as defined in the appended claims.

## Claims

1. Apparatus for endoscopy, comprising: an endoscope-adapted garment (20) having an aperture (28) sized and located to permit insertion of an endoscope (40) therethrough into a body orifice of a patient (22) while the garment (20) is worn by the patient (22); and an endoscope retaining device (42), which is fastened within the aperture (28) so as to hold the endoscope (40) in place when the endoscope (40) is inserted into the body orifice.

2. The apparatus according to claim 1, wherein the endoscope retaining device (42) comprises a rigid material having a bore (46) therethrough, which is sized to fit snugly around the endoscope (40), and wherein the garment (20) comprises a fastening assembly (44) for fixing the retaining device (42) to the garment (20).

3. The apparatus according to claim 1, wherein the aperture (28) in the garment (20) is sized and located to permit insertion of the endoscope (40) into an anus of the patient (22).

4. The apparatus as claimed in any preceding claim, the garment (20) comprising a fabric (24), which is cut and sized to fit snugly around a pelvic area of the patient (22), such that when the garment (20) is worn by the patient (22), the fabric defines two leg holes (26) and said aperture (28), which is positioned adjacent to a body orifice of the patient (22) so as to permit insertion of an endoscope (40) through the aperture (28) into the body orifice.

5. The apparatus according to claim 4, wherein the fabric (24) comprises a sheet of material having front and rear portions joined by a central portion, which is cut to fit between the legs of the patient (22), and wherein the garment (20) further comprises one or more fasteners (30), which are adapted to fix the front and rear portions together so as to cover the pelvic area.

6. The apparatus according to claim 5, wherein the aperture (28) in the garment (20) is sized and located to permit insertion of the endoscope (40) into an anus of the patient (22).

7. An endoscopy system comprising: an endoscope (40) adapted to be inserted into a body orifice of a patient (22); and an apparatus as claimed in any preceding claim.

8. The system according to claim 8, wherein the endoscope (40) is adapted to be inserted into an anus of the patient (22).

## Patentansprüche

1. Endoskopiegerät, welches enthält: ein zur Endoskopie ausgelegtes Kleidungsstück (20), welches eine Apertur (28), welche derart bemessen und positioniert ist, um ein Einsetzen von einem Endoskop (40) **dadurch** in eine Körperöffnung von einem Patienten (22) zuzulassen, während das Kleidungsstück (20) von dem Patienten (22) getragen wird, und eine Endoskop-Haltevorrichtung (42) hat, welche innerhalb der Apertur (28) befestigt ist, um somit das Endoskop (40) an Ort und Stelle zu halten, wenn das Endoskop (40) in die Körperöffnung eingesetzt ist.

2. Gerät nach Anspruch 1, bei welchem die Endoskop-Haltevorrichtung (42) ein starres Material enthält, welches eine Bohrung (46) **dadurch** hat, welche derart bemessen ist, dass sie gleichmäßig um das Endoskop (40) herum passt, und wobei das Kleidungsstück (20) eine Befestigungsanordnung (44) enthält, um die Haltevorrichtung (42) an dem Kleidungsstück (20) zu befestigen.

3. Gerät nach Anspruch 1, bei welchem die Apertur (28) in dem Kleidungsstück (20) derart bemessen und positioniert ist, um ein Einsetzen des Endoskops (40) in einen Anus des Patienten (22) zu erlauben.

4. Gerät nach einem der vorangehenden Ansprüche, bei welchem das Kleidungsstück (20) einen Stoff (24) enthält, welcher derart ausgeschnitten und bemessen ist, um gleichmäßig um einen Beckenbereich von dem Patienten (22) zu passen, so dass, wenn das Kleidungsstück (20) von dem Patienten (22) getragen wird, der Stoff zwei Beinlöcher (26) und die Apertur (28) bestimmt, welche angrenzend zu einer Körperöffnung von dem Patienten (22) positioniert ist, um somit ein Einsetzen von einem Endoskop (40) durch die Apertur (28) in die Körperöffnung zu erlauben.

5. Gerät nach Anspruch 4, bei welchem der Stoff (24) eine Materialbahn enthält, welche einen vorderen und einen hinteren Abschnitt hat, welche an einem mittigen Abschnitt verbunden sind, welche derart ausgeschnitten ist, um zwischen den Beinen von dem Patienten (22) zu passen, und wobei das Kleidungsstück (20) ferner eine oder mehrere Befestigungseinrichtungen (30) enthält, welche dazu ausgelegt sind, um den vorderen und hinteren Abschnitt zusammen zu fixieren, um somit den Beckenbereich zu bedecken.

6. Gerät nach Anspruch 5, bei welchem die Apertur (28) in dem Kleidungsstück (20) derart bemessen und positioniert ist, um ein Einsetzen des Endoskops (40) in einen Anus von dem Patienten (22) zu ermöglichen.

7. Endoskopiesystem, welches enthält: ein Endoskop (40), welches dazu ausgelegt ist, um in eine Körperöffnung von einem Patienten (22) eingesetzt zu werden, und ein Gerät nach einem der vorangehenden Ansprüche.

8. System nach Anspruch 7, bei welchem das Endoskop (40) dazu ausgelegt ist, um in einen Anus von dem Patienten (22) eingesetzt zu werden.

## Revendications

1. Appareil pour une endoscopie, comportant : un vêtement adapté à un endoscope (20) ayant une ouverture (28) dimensionnée et positionnée pour permettre l'insertion d'un endoscope (40) à travers celle-ci jusque dans un orifice corporel d'un patient (22) tandis que le vêtement (20) est porté par le patient (22), et un dispositif de retenue d'endoscope (42), qui est fixé dans l'ouverture (28) de manière à maintenir l'endoscope (40) en place lorsque l'endoscope (40) est inséré dans l'orifice corporel.

2. Dispositif selon la revendication 1, dans lequel le dispositif de retenue d'endoscope (42) est constitué d'un matériau rigide ayant un alésage (46) à travers celui-ci, qui est dimensionné pour s'agencer de manière serrée autour de l'endoscope (40), et dans lequel le vêtement (20) comporte un ensemble de fixation (44) pour fixer le dispositif de retenue (42) sur le vêtement (20).

3. Dispositif selon la revendication 1, dans lequel l'ouverture (28) dans le vêtement (20) est dimensionnée et positionnée pour permettre l'insertion de l'endoscope (40) dans l'anus du patient (22).

4. Dispositif selon l'une quelconque des revendications précédentes, le vêtement (20) comportant un tissu (24), qui est découpé et dimensionné pour s'agencer de manière serrée autour d'une zone pelvienne du patient (22), de telle sorte que lorsque le vêtement (20) est porté par le patient (22), le tissu défini deux trous de jambe (26) et ladite ouverture (28) qui est positionnée adjacente à un orifice corporel du patient (22) de manière à permettre l'insertion d'un endoscope (40) à travers l'ouverture (28) jusque dans l'orifice corporel.

5. Dispositif selon la revendication 4, dans lequel le tissu (24) comporte une feuille de matériau ayant des parties avant et arrière reliées par une partie centrale, qui est découpée pour s'agencer entre les jambes du patient (22), et dans lequel le vêtement comporte en outre une ou plusieurs fixations (30) qui sont adaptées pour fixer les parties avant et arrière ensemble de manière à recouvrir la zone pelvienne.

6. Dispositif selon la revendication 5, dans lequel l'ouverture (28) située dans le vêtement (5) est dimensionnée et positionnée pour permettre l'insertion de l'endoscope (40) dans l'anus du patient (22).

7. Système d'endoscopie, comportant : un endoscope (40) adapté pour être inséré dans l'orifice corporel d'un patient (22), et un dispositif selon l'une quelconque des revendications précédentes.

8. Système selon la revendication 8, dans lequel l'endoscope (40) est adapté pour être inséré dans l'anus du patient (22).
